Europäisches Patentamt

European Patent Office

Office européen des brevets

(19)

(11) Publication number: **0 335 135**
**A2**

(12) **EUROPEAN PATENT APPLICATION**

(21) Application number: **89103860.6**

(22) Date of filing: **06.03.89**

(51) Int. Cl.⁴: **C12P 21/00 , A61K 39/29 , C12N 5/00 , G01N 33/576**

(30) Priority: **30.03.88 US 175068**

(43) Date of publication of application:
**04.10.89 Bulletin 89/40**

(84) Designated Contracting States:
**AT BE CH DE ES FR GB IT LI NL**

(71) Applicant: **ABBOTT LABORATORIES**
**One Abbott Park Road**
**Abbott Park, IL 60064-3500(US)**

(72) Inventor: **Carrick, Robert J.**
**9925 4th Avenue**
**Kenosha Wisconsin 53140(US)**
Inventor: **Linke, Hawley K.**
**1955 Linden Avenue**
**Highland Park Illinois 60035(US)**
Inventor: **Lesniewski, Richard R.**
**8706 110th Avenue**
**Kenosha Wisconsin 53142(US)**
Inventor: **Peterson, David A.**
**6134 218th Avenue**
**Bristol Wisconsin 53104(US)**

(74) Representative: **Modiano, Guido et al**
**MODIANO, JOSIF, PISANTY & STAUB**
**Modiano & Associati Via Meravigli, 16**
**I-20123 Milan(IT)**

(54) **Production of monoclonal antibodies specific for non-A, non-B hepatitis infected liver.**

(57) The present invention relates to antigens specifically associated with parenterally transmitted non-A, non-B hepatitis (NANB) and to monoclonal antibodies capable of selectively binding with antigens associated with parenterally transmitted NANB hepatitis. The present invention also provides for a cell line capable of producing a monoclonal antibody which selectively binds with antigens specifically associated with parenterally transmitted NANB hepatitis infection and for an immunological procedure for detecting agents specifically associated with parenterally transmitted NANB hepatitis on the basis of a selective immunological reaction with an antibody specific therefor.

# PRODUCTION OF MONOCLONAL ANTIBODIES SPECIFIC FOR NON-A, NON-B HEPATITIS INFECTED LIVER

## BACKGROUND OF THE INVENTION

The present invention pertains to antigens associated with parenterally transmitted non-A, non-B hepatitis (NANB hepatitis) and to monoclonal antibodies capable of specifically binding with these antigens.

Acute viral hepatitis is diagnosed based on the results of routine biochemical laboratory tests conducted when a patient presents clinically with a well defined set of physical findings including, e.g., jaundice, hepatic tenderness, etc. Serologically, acute viral hepatitis is accompanied by marked elevation in levels of alanine aminotransferase and aspartate aminotransferase, with only minimal elevation in levels of alkaline phosphatase and lactic acid dehydrogenase. Additional specific serologic immunoassays are frequently performed to determine whether the acute viral hepatitis is the result of infection by a type A, B, or delta (D) viral causative agent. If these and additional tests for Epstein-Barr virus and cytomegalovirus are negative, the condition is diagnosed as NANB hepatitis by default. That is, as noted in Hellings, J.A., Vox Sang, 51: (Suppl. 1) 63-66 (1986), NANB hepatitis is characterized by what it is not rather than by providing convincing evidence of the isolation of or the demonstration of specific antibodies to the etiologic agent(s) responsible for blood and blood product borne (i.e., parenterally transmitted) NANB hepatitis.

Each of the immunologically characterized hepatitis-inducing viruses [hepatitis A virus (HAV), hepatitis B virus (HBV) and hepatitis D virus (HDV)] belongs to a separate family of viruses, and has a distinctive viral organization, protein structure and mode of replication. Attempts to identify the NANB hepatitis virus by virtue of genomic similarity to one of the known hepatitis viruses have failed, suggesting that NANB also has a distinct organization and structure. [Fowler, et al., J. Med. Virol., 12:205-213 (1983) and Weiner, et al., J. Med. Virol., 21:239-247 (1987)].

Progress in developing and characterizing an antibody preparation specific for NANB hepatitis has been particularly hampered by difficulties in correctly identifying antigen(s) associated with NANB hepatitis. There have been many initial "identifications" of parenterally transmitted NANB and associated antigens which have proved erroneous. For example, Wands, J., et al., in PCT/US83/01412 (published March 29, 1984 as WO 8401107) describe the identification, isolation, characterization, purification, and use of NANB hepatitis virus believed to be an attenuated or inactivated form of a DNA virus. Although the authors claimed to have distinguished the NANB hepatitis virus from HAV and HBV, in fact, the monoclonal antibodies used to characterize and detect the alleged NANB virus were, in addition, cross-reactive with HBV antigen. See also, Wands, et al., Proc. Nat'l. Acad. Sci., 83:6608-6612 (1986), wherein it was demonstrated that the viral samples of the Wands, et al. published application contained a viral nucleic acid sequence identical to HBV in length and capable of inducing HBV anti-core immunologic responses.

As another example, Ohori, H., et al., Japanese Patent No. Sho 60[1985]-89430, described a NANB hepatitis associated antigen, referred to as the SO-antigen, capable of inducing strong agglutination reactions with the serum and urine of NANB hepatitis patients but not with normal serum or with serum from HBV patients. However, no samples from any other types of hepatitis, such as HDV, were tested. In a related publication, Ohori, et al., J. Med. Virol, 12:161-178 (1983) the SO-antigen was characterized as being associated with an epidemic form of NANB hepatitis rather than with parenterally transmitted NANB hepatitis. Recent reports [summarized by Bradley, et al., Proc. Nat'l. Acad. Sci., 84:6277-6281, (1987)] have additionally established that the epidemic form, designated as enterically-transmitted NANB (ET-NANB), is a new form of viral hepatitis with an enteric route of natural infection in humans. ET-NANB is unrelated to parenterally transmitted NANB.

A number of workers believed they had correctly identified an antigen, designated as AN6520, as specifically associated with NANB hepatitis. Akatsuka, T., et al., in European Patent Application No. 0 092 249, published October 26, 1983, described a NANB hepatitis-associated monoclonal antibody, a culture composition, and a diagnostic reagent containing the same. Because the antigen did not react with a variety of hepatitis associated antibodies: HAV antibody, anti-HBs, anti-HBc, anti-HBe, anti-delta, nor normal human liver supernatant antibody or various normal human plasma antibodies, the antigen was believed to be a specific antigen associated with NANB hepatitis. Subsequently, Tohmatsu, J., et al., J. Med. Virol., 15:357-371 (1985), described the purification of AN6520 antigen from liver infected with NANB hepatitis virus and Akatsuka, T., et al., J. Med. Virol, 20:33-42 (1986), described the detection of AN6520. However, in Akatsuka, T., et al., J. Med. Virol, 20:43-56 (1986), AN6520 was shown to be a protein found in normal, uninfected livers as well as in NANB infected livers. Accordingly, monoclonal antibodies generated utilizing AN6520 antigen are not useful for confirming the presence of NANB hepatitis.

There have been other references relating to monoclonal antibodies believed to have specificity exclusively for NANB hepatitis but later found to display cross-reactivity with HDV. Replication of HDV appears dependent on simultaneous infection with HBV (or in experimental animal models of other hepadna viruses). In vivo, HDV infections share some identifiable characteristics with NANB hepatitis infections. Examination of HDV and NANB hepatitis infected livers by light and electron microscopy shows similar structural anomalies not observed in normal livers nor in those with other forms of hepatitis. A monoclonal antibody originally described as specific for NANB hepatitis infected liver (Shimizu, Y., et al., Proc. Nat'l. Acad. Sci. (USA), 82/7:2138-2142 (1985)) has been shown to recognize one component of these structural anomalies in both HDV and NANB hepatitis infected liver. While initial results disclosed continuous cell lines of chimpanzee and human lymphocytes producing antibodies specifically associated with NANB hepatitis, subsequent studies demonstrated that the antibodies designated 48-1 and S-1 also reacted with HDV infected livers. See, Shimizu, Y.K., et al., Hepatology (United States), 6:1329-33 (1986); also Ono, Y., et al., European Patent Application No. 0 190 972 published August 13, 1986, and two corresponding Japanese applications JP 61056196 and JP 61025484 by the co-authors of the Shimizu, et al. publications).

To date, no antigen nor antibody specific for NANB hepatitis has been unambiguously demonstrated. Accordingly, there exists a need in the art for an antibody reactive exclusively with NANB infected liver tissue.

## BRIEF SUMMARY OF THE INVENTION

The present invention relates to antigens specifically associated with parenterally transmitted NANB hepatitis infection and to antibodies, particularly monoclonal antibodies, capable of selectively binding with such antigens.

Provided by the invention are cell lines producing monoclonal antibodies capable of selectively binding with antigens associated with parenterally transmitted NANB hepatitis infection. Such antibodies are highly useful in immunological procedures for detecting parenterally transmitted NANB hepatitis infection. "Selective" as used herein with reference to immunobinding with antigens associated with NANB hepatitis shall refer to the capacity to immunobind said antigens to the exclusion of antigens associated with HBV-infected, HDV-infected and normal liver tissue as determined, for example, by the immunofluorescent staining procedures illustrated in Example 2 hereof. Correspondingly, "specifically associated" as used herein with reference to NANB hepatitis antigens shall refer to those antigens specifically associated with NANB hepatitis to the exclusion of (i.e., having no epitopes in common with) antigens associated with HBV-infected, HDV-infected, and normal liver tissue.

Presently preferred monoclonal antibodies include those derived from cells of NANB hepatitis infected chimpanzees. Particularly preferred is an IgM antibody, designated "aPt-1", which is obtained from the supernatant of cultured growth of cell line aPt-1, an EBV-transformed chimpanzee lymphocyte cell line of the invention. This cell line was deposited on March 29, 1988 with the American Type Culture Collection, 12301 Parklawn Drive, Rockville, Maryland, 20852 under accession No. CRL 9679.

Other aspects and advantages of the present invention will be apparent upon consideration of the following detailed description thereof which includes numerous illustrative examples of the practice of the invention.

## DETAILED DESCRIPTION

The following examples illustrate practice of the invention in the isolation of lymphocytes from NANB hepatitis infected chimpanzees for the preparation of EBV-transformed antibody producing cell lines, the production of monoclonal antibodies capable of selective binding with antigens specifically associated with NANB hepatitis infection, and in the identification of antigens associated with NANB hepatitis infection.

More specifically, Example 1 relates to NANB hepatitis infection procedures and the preparation of liver biopsy specimens for use in histological screening procedures. Example 2 relates to preparation and immunological screening of lymphocyte derived cell lines and to immunofluorescence and immunoglobulin assays used to detect monoclonal antibodies specific for NANB hepatitis.

The examples which follow are for illustrative purposes only and are not intended in any way to limit the scope of the invention.

## EXAMPLE 1

### Liver Biopsy Specimens:

All chimpanzees (Pan troglodytes) studied were housed at the Laboratory for Experimental Medicine and Surgery in Primates (LEMSIP), Tuxedo, New York. Surgical liver biopsy (normal) tissue was obtained from two animals prior to inoculation with NANB liver homogenates. During the acute phase of the ensuing disease, as indicated by cytoplasmic ultrastructural changes of the hepatocytes [Pfeifer, et al., Cell Pathology, 33:233-243 (1980)] and increased serum levels of liver enzymes (especially alanine aminotransferase-ALT), a second (acute) surgical biopsy was performed. A homogenate prepared from the acute biopsy of one animal was used to inoculate the second animal.

Additional liver biopsy specimens were obtained from the following chimpanzees i) a chronic HBV carrier; ii) a chimpanzee who succumbed bearing an NANB-associated hepatocellular carcinoma [Linke, et al., pp. 357-370, In Hepadna Viruses, Robinson W., et al., (eds): Alan R. Liss, New York (1987); Muchmore, et al., J. Med. Virol., 21:49A (1987)]; and iii) a chronic HBV carrier with an HDV superinfection. Biopsy material was obtained from this third chimpanzee when serum levels of delta antigen were the greatest. A human liver infected with HDV was obtained following autopsy. Tissues from all chimpanzee biopsies were sliced and frozen in liquid nitrogen immediately upon removal from the animal.

## EXAMPLE 2

### Preparation of Lymphocyte Derived Cell Lines:

Whole blood was obtained from a chimpanzee at a time when he was convalescent from an NANB infection but still chronically infected with HBV. Lymphocytes were purified with Ficol-Paque (Pharmacia, Piscataway, NJ) according to the manufacturer's instructions and infected with Epstein-Barr virus (EBV) secreted from B95-8 cells [Miller, et al., Proc. Nat'l. Acad. of Sci. USA, 70:190-194 (1973), essentially as described in VanMeel, et al., J. of Immuno. Methods, 80:267-276, (1985)]. Cells were seeded 1 to 4 x 10⁴ per microtiter well in RPMI medium containing 20% fetal calf serum (FCS). Supernatant fluids from all wells were subjected to initial analysis by immunofluorescence staining before transfer of the cells to larger vessels. After transfer, cell lines were adapted for growth in decreasing FCS concentrations (down to 1%) and 1% Nutradoma-Hu (Boehringer Mannheim Biochemicals, Indianapolis, IN).

### Immunofluorescence and Immunoglobulin Assays:

Cell culture supernatants, prepared according to Example 1, from 1,440 individual microtiter wells were assayed by immunofluorescence for immunoreactivity against acute NANB infected liver sections according to the following procedure. Cryostat sections of frozen liver, 4 microns thick, were applied to microscope coverslips pre-coated with bovine serum albumin. Sections were incubated with undiluted cell culture medium for six hours at room temperature. After rinsing with phosphate buffered saline (PBS), sections were incubated with dilute fluorescein-conjugated IgG (caprine) directed against human IgM and IgG overnight at 4°C. Sections were rinsed and mounted in 50% glycerol/PBS prior to evaluation by fluorescence microscopy. Of these, 421 (29%) gave positive signals and were re-screened against normal and against NANB liver sections. This screening was completed after a total time in culture of three months. Sixty-two supernatants (4% of the original 1,440) displayed persistent and differential signals.

An additional re-screening compared sections of HBV-infected chimpanzee liver to sections of normal and of NANB-infected specimens. Twenty-two of the 62 cell supernatants gave positive signals only against the sections of NANB liver. Two of the twenty-two cell culture supernatants contained both IgG and IgM antibodies while the remainder exhibited only IgM activity.

4

Immunoglobulin levels present in tissue culture supernatants were determined using a solid phase ELISA. Briefly, commercially available affinity purified anti-human IgG or IgM (caprine) was adsorbed to 1/4 inch diameter polystyrene beads. Fifty $\mu$L of diluted culture supernatants plus 200 $\mu$L of an appropriate diluent were incubated with the solid phase for 1 hour at 40°C. The beads were washed and 200 $\mu$1 of anti-human IgG or IgM coupled to horseradish peroxidase were added and incubated for 2 hours at 40°C. Beads were again washed and the chromogenic substrate, o-phenylenediamine, was added. Color development was monitored at 492 nm. Standard curves produced using commercially available human IgG or IgM diluted in the growth media were linear through the concentration range of interest. Concentrations of immunoglobulins in the EBV-transformed culture supernatants were determined from these standard curves.

After six months in culture, only 13 IgM producing cells lines remained stable. The designations of the monoclonal-like antibodies produced by these cell lines and their respective staining patterns throughout all assays are listed in Table 1.

TABLE 1

| Characteristics of Immunofluorescent Staining | | | | | |
|---|---|---|---|---|---|
| Monoclonal Designation | Description of Staining Observed | Liver disease* | | | |
| | | Normal | HBV | NANB | HDV |
| aPt-1 | punctate cytoplasmic | - | - | + | - |
| aPt-2 | surface and cytoplasmic | - | - | + | + |
| aPt-3 | strong nuclear (poor specificity) | - | - | + | + |
| aPt-4 | punctate cytoplasmic | - | - | + | + |
| aPt-5 | perinuclear with rare cytoplasmic | - | - | + | + |
| aPt-6 | perinuclear with grape-like clusters | - | - | + | +/- |
| aPt-7 | pinpoint cytoplasmic | - | - | + | + |
| aPt-8 | membrane-associated | - | - | + | +/- |
| aPt-9 | diffuse and punctate cytoplasmic | - | - | + | + |
| aPt-10 | punctate cytoplasmic | - | - | + | + |
| aPt-11 | diffuse cytoplasmic | - | - | + | + |
| aPt-12 | nuclear and cytoplasmic | - | - | + | + |
| aPt-13 | bleb-like, membrane-associated | - | - | + | na |

* HBV - Hepatitis B Virus
NANB - Non-A, Non-B Hepatitis
HDV - Hepatitis Delta Virus
- - no specific immunofluorescent staining observed.
+ - strong, positive immunofluorescent stain.
+/- - indeterminant or weak immunofluorescent staining.
na - not assayed.

Media from 12 of the 13 stable aPt cell lines were re-assayed against normal, NANB infected, as well as both the human and the chimpanzee HDV infected livers. As shown in Table 1, all but one monoclonal antibody (aPt-1) of the 12 monoclonal antibodies assayed reacted with the HDV infected tissues. The 12 monoclonal antibodies display more than one staining pattern, possibly because they represent reactivity against different antigens expressed in common between NANB and HDV infected liver.

The monoclonal antibody produced by cell line aPt-1 demonstrates a punctate cytoplasmic staining on both NANB acute liver sections and on sections obtained from the NANB-associated hepatocellular carcinoma. No positive fluorescence is observed from the normal, HBV or HDV infected livers. Thus, in this example, only monoclonal antibody aPt-1 is truly specific for detecting NANB infection.

In addition, preliminary studies are underway to evaluate the specificity of aPt-1 on human liver sections. These liver specimens include those obtained from normal liver, HBV liver, HBV/HDV liver, NANB liver, alcoholic disease liver, and autoimmune disease liver.

It is expected that monoclonal antibodies according to the invention will be useful in assays for detecting parenterally transmitted NANB hepatitis infection and infectious agents in bodily fluids, tissues, experimental reagents and fluids proposed for parenteral administration.

The monoclonal antibodies of the invention may also be used to isolate antigen specifically associated with NANB hepatitis, e.g., by affinity chromatography. Such immunopurified antigen can be used to immunize, e.g., non-primate species such as goats and rabbits, in order to obtain antibodies having multiple epitope specificities. Alternatively, the isolated antigen can be used to immunize mice or rats allowing for preparation of hybridoma cell lines capable of producing monoclonal antibodies.

Antibodies provided according to the present invention will make possible the characterization of antigens specifically associated with NANB hepatitis infection and infectious agents. In this regard, standard methods such as analytical and preparative gel electrophoresis may be employed to determine the molecular weight of antigens reactive with aPt-1. Reactive isolates so obtained may be subjected to further characterization through use of various selective agents including reducing agents, proteases, lipases, glycosidases, and the like. Thus, also contemplated are assays for NANB-associated antibodies based on use of isolated antigens and antigen fragments provided by the invention. Assays of the invention may be provided in a variety of configurations including competitive and non-competitive immunoassays, radioimmunoassays, ELISA's, FIA's and the like.

While the present invention has been illustrated in terms of specific methods and compositions, it is understood that variations and modifications will occur to those skilled in the art upon consideration of the present invention.

For example, it is envisioned that immortalized lymphocytes obtained from various chimpanzees, humans, or other mammals which have been exposed to the agent for NANB hepatitis will also be effective to provide antibodies of IgM and IgG isotypes according to the present invention. Although the preferred monoclonal producing cell lines are those immortalized by the infection with Epstein-Barr virus; it is not intended to preclude others which may be maintained by additional approaches to monoclonal antibody production stabilization such as cell fusion or any other effective means known to those skilled in the art from being included in the scope of the invention. It is also envisioned that additional monoclonal antibodies produced according to the present invention will be capable of identifying more than one non-A, non-B specific epitope and/or antigen.

## Claims

1. An antibody capable of selective immunobinding with one or more antigens specifically associated with parenterally transmitted NANB hepatitis.

2. The antibody according to claim 1, produced by cell line aPt-1 (ATCC No. CRL 9676).

3. Antigens specifically associated with parenterally transmitted NANB hepatitis and characterized by selective immunobinding with an antibody of claim 1.

4. A cell line capable of producing an antibody capable of selectively binding with one or more antigens specifically associated with parenterally transmitted NANB hepatitis.

5. The cell line according to claim 4, which is cell line aPt-1 (ATCC No. CRL 9676).

6. In an immunological assay for detecting antigens specifically associated with parenterally transmitted NANB hepatitis on the basis of selective immunological reaction with an antibody specific therefor, the improvement comprising:
employing an antibody of claims 1 or 2.